# EUROPEAN PATENT APPLICATION

(11) **EP 4 800 076 A1**
(43) Date of publication of application: **02.09.2026**
(21) Application number: 24882427.8
(22) Date of filing: 23.10.2024
(51) Int. Cl.: C08L 101/02, A61L 24/00, A61L 24/08, A61L 24/10, C07K 14/78, C07K 19/00, C08L 3/02, C08L 5/00, C08L 77/04

(54) **COMPOSITION**

(30) Priority: 24.10.2023 JP 2023182291
(71) Applicant: NATIONAL UNIVERSITY CORPORATION TOKAI NATIONAL HIGHER EDUCATION AND RESEARCH SYSTEM, Nagoya-shi, Aichi 464-8601 (JP)
(72) Inventor: NARUTAKI, Ayae, Nagoya-shi, Aichi 464-8601 (JP); KANDA, Mitsuro, Nagoya-shi, Aichi 464-8601 (JP); OHTSUKI, Chikara, Nagoya-shi, Aichi 464-8601 (JP); SUZUKI, Kazumasa, Nagoya-shi, Aichi 464-8601 (JP); SHINOZUKA, Takahiro, Nagoya-shi, Aichi 464-8601 (JP); AOYAMA, Yuki, Nagoya-shi, Aichi 464-8601 (JP)
(74) Representative: Hoffmann Eitle
(86) International application number: PCT/JP2024/037836
(87) International publication number: WO 2025/089316

(57) **Abstract**

Provided is a material that is obtained by using an elastin-like polypeptide and that has higher strength and/or bioadhesive properties after self-healing. Specifically provided is a composition comprising a polypeptide that comprises an elastin-like block peptide sequence and has an amino group, the elastin-like block peptide sequence comprising a G sequence block consisting of (SEQ ID NO: 1: X¹GGX²G)ₙ, wherein each X¹ is the same or different, and represents V or L, each X² is the same or different, and represents V or L, and n represents an integer of 4 or more, and a P sequence block consisting of (SEQ ID NO: 2: VPGX³G)ₘ, wherein each X³ is the same or different, and represents any amino acid, and m represents an integer of 5 or more, and comprising a water-soluble polymer having an aldehyde group.

## Description

### Technical Field

The present invention relates to a composition usable for medical devices for use in, for example, protecting an internal sutured site, a cut surface of an organ or a tissue, or a damaged site.

### Background Art

In gastrointestinal surgery, anastomotic leakage, in which the sutured site undergoes disruption after surgery, resulting in leakage of digestive juices, is a problem. It has been reported that anastomotic leakage occurs with a probability of 4 to 21%, which in some cases causes peritonitis and may lead to death. Additionally, at a cut surface formed by organ resection, such as pancreatectomy or hepatectomy, leakage of body fluids, such as blood and tissue fluid, occurs. Therefore, it is necessary to protect the sutured site and the cut surface with some kind of material.

Fibrin glue is sometimes used as a protective material. Fibrin glue is a paste-like substance prepared by mixing fibrinogen with a plurality of agents, such as thrombin, and is used by being applied to a target site in liquid form. However, plasma is used as a raw material in the production of fibrin glue, and there are thus concerns regarding infection risks and instability in the supply of raw materials.

A non-woven fabric obtained by using polyglycolic acid as a material is also sometimes used as a protective material (NPL 1). However, since this is not liquid, the shape cannot be freely deformed according to the target to be protected.

### Citation List

### Non-patent Literature

NPL 1: Journal of the American College of Surgeons, 2016; 222(1): 59-64
NPL 2: International Journal of Molecular Sciences, 22, 4104 (12 pages) (2021)

### Summary of Invention

### Technical Problem

NPL 2 reports that an elastin-like polypeptide produced by simplifying the amino acid sequence of elastin self-assembles into nanofibers in water when the temperature is increased, thereby forming a hydrogel, and that the gel exhibits self-healing (thixotropic) properties, in which it liquefies upon shaking and re-forms a gel upon standing. The present inventors considered the use of such an elastin-like polypeptide, with the expectation of creating a material that can be applied to a target to be protected in liquid form and that can promote healing while reinforcing a target site to be protected by gelation. However, the strength and bioadhesive properties after self-healing of the gel composed of the elastin-like polypeptide alone were insufficient.

An object of the present invention is to provide a material that is obtained by using an elastin-like polypeptide and that exhibits higher strength and/or bioadhesive properties after self-healing.

### Solution to Problem

The present inventors conducted extensive research to achieve the above object and found that the object can be achieved by a composition comprising a polypeptide that comprises an elastin-like block peptide sequence and has an amino group, the elastin-like block peptide sequence comprising a G sequence block consisting of (SEQ ID NO: 1: X¹GGX²G)ₙ, wherein each X¹ is the same or different, and represents V or L, each X² is the same or different, and represents V or L, and n represents an integer of 4 or more, and a P sequence block consisting of (SEQ ID NO: 2: VPGX³G)ₘ, wherein each X³ is the same or different, and represents any amino acid, and m represents an integer of 5 or more, and comprising a water-soluble polymer having an aldehyde group. The inventors conducted further research on the basis of this finding and completed the present invention. Specifically, the present invention includes the following subject matter.

Item 1. A composition comprising:
a polypeptide that comprises an elastin-like block peptide sequence and has an amino group, the elastin-like block peptide sequence comprising a G sequence block consisting of (SEQ ID NO: 1: X¹GGX²G)ₙ, wherein each X¹ is the same or different, and represents V or L, each X² is the same or different, and represents V or L, and n represents an integer of 4 or more, and a P sequence block consisting of (SEQ ID NO: 2: VPGX³G)ₘ, wherein each X³ is the same or different and represents any amino acid, and m represents an integer of 5 or more; and
a water-soluble polymer having an aldehyde group.

Item 2. The composition according to Item 1, wherein the composition is a gel composition or a gel-forming sol composition.

Item 3. The composition according to Item 1, wherein the polypeptide comprises an amino acid residue having an amino group.

Item 4. The composition according to Item 3, wherein the polypeptide comprises a crosslinking sequence block containing two or more amino acid residues each having an amino group.

Item 5. The composition according to Item 1, wherein the polypeptide comprises a cell adhesive sequence block.

Item 6. The composition according to Item 1, wherein the water-soluble polymer comprises a sugar as a structural unit.

Item 7. The composition according to Item 6, wherein the water-soluble polymer is an oxidized polysaccharide.

Item 8. The composition according to Item 6, wherein the water-soluble polymer is oxidized dextran and/or oxidized dextrin.

Item 9. The composition according to Item 1, wherein the composition comprises a conjugate of the polypeptide and the water-soluble polymer linked via a covalent bond.

Item 10. A method for producing the composition according to any one of Items 1 to 9, comprising:
(a) mixing a sol composition converted from a gel composition with a water-soluble polymer having an aldehyde group, the gel composition comprising a polypeptide that comprises an elastin-like block peptide sequence and has an amino group, the elastin-like block peptide sequence comprising a G sequence block consisting of (SEQ ID NO: 1: X¹GGX²G)ₙ, wherein each X¹ is the same or different, and represents V or L, each X² is the same or different, and represents V or L, and n represents an integer of 4 or more, and a P sequence block consisting of (SEQ ID NO: 2: VPGX³G)ₘ, wherein each X³ is the same or different, and represents any amino acid, and m represents an integer of 5 or more.

Item 11. A medical device comprising the composition according to any one of Items 1 to 9.

Item 12. The medical device according to Item 11 for use in protecting or adhering to a sutured site, a cut surface of an organ or tissue, or a damaged site.

### Advantageous Effects of Invention

The present invention can provide a material that is obtained by using an elastin-like polypeptide and that exhibits higher strength and/or bioadhesive properties after self-healing.

### Brief Description of Drawings

Fig. 1 shows schematic views of an aluminum test specimen used for the lap shear measurement in Section 1.4.4.
Fig. 2 shows the results of the FTIR spectroscopic measurement in Section 2.1.1.
Fig. 3 shows the results of SDS-PAGE in Section 2.2.1.
Fig. 4 shows the results of the NMR spectroscopic measurement in Section 2.2.2.
Fig. 5 shows the results of the recovery behavior test in Section 2.2.3.
Fig. 6 shows the results of the lap shear measurement in Section 2.2.4.
Fig. 7 shows the results of the in vivo gelation test in Section 2.2.5.

### Description of Embodiments

In the present specification, the terms "comprise," "contain," and "include" include the concepts of "comprising," "containing," "consisting essentially of," and "consisting of."

In the present specification, amino acid residues in an amino acid sequence may be simply referred to as "amino acids" or by their specific amino acid names (e.g., valine and leucine). Amino acid residues in an amino acid sequence may also be represented by single-letter amino acid codes.

In one aspect, the present invention relates to a composition comprising a polypeptide that comprises an elastin-like block peptide sequence and has an amino group, the elastin-like block peptide sequence comprising a G sequence block consisting of (SEQ ID NO: 1: X¹GGX²G)ₙ, wherein each X¹ is the same or different, and represents V or L, each X² is the same or different, and represents V or L, and n represents an integer of 4 or more, and a P sequence block consisting of (SEQ ID NO: 2: VPGX³G)ₘ, wherein each X³ is the same or different, and represents any amino acid, and m represents an integer of 5 or more (the peptide may be referred to as "the peptide of the present invention" in the present specification), and comprising a water-soluble polymer having an aldehyde group (the composition may be referred to as "the composition of the present invention" in the present specification). This is described below.

The G sequence block is not particularly limited as long as it is a block consisting of a repeat sequence (X¹GGX²G)ₙ of the amino acid sequence (X¹GGX²G) set forth in SEQ ID NO: 1. The G sequence block is typically a block that can take a β-sheet structure. The G sequence block can impart the ability to form fibers by self-assembly to the polypeptide.

Each X¹ is the same or different, and represents V or L, and each X² is the same or different, and represents V or L. X¹ and X² are preferably V.
n represents an integer of 4 or more. When n is 4 or more, the polypeptide of the present invention can form a fibrous self-assembly. n is preferably 4 to 20, more preferably 4 to 12, even more preferably 4 to 8, and still even more preferably 4 to 6.

The P sequence block is not particularly limited as long as it is a block consisting of a repeat sequence (VPGX³G)ₘ of the amino acid sequence (VPGX³G) set forth in SEQ ID NO: 2. The P sequence block is typically a block that can take a β-turn structure. The P sequence block allows the polypeptide to have a lower critical solution temperature (LCST) and to self-assemble at the LCST or higher to phase separate from an aqueous solution.

The LCST of the polypeptide of the present invention (solvent: water, concentration: 0.03 wt%) is, for example, 10 to 30°C, and preferably 15 to 25°C.

Each X³ is the same or different, and represents any amino acid. Examples of amino acids represented by X³ include amino acids having a basic side chain, such as lysine, arginine, and histidine; amino acids having an acidic side chain, such as aspartic acid and glutamic acid; amino acids having an uncharged polar side chain, such as glycine, asparagine, glutamine, serine, threonine, tyrosine, and cysteine; amino acids having a nonpolar side chain, such as alanine, valine, leucine, isoleucine, proline, phenylalanine, methionine, and tryptophan; amino acids having a β-branched side chain, such as threonine, valine, and isoleucine; and amino acids having an aromatic side chain, such as tyrosine, phenylalanine, tryptophan, and histidine. The amino acids represented by X³ are preferably amino acids other than proline.

The LCST can be adjusted by the amino acids represented by X³. If the amino acids represented by X³ are hydrophilic amino acids, the LCST becomes too high, which is not preferable. From the viewpoint of easily adjusting the LCST to an appropriate (physiologically acceptable) temperature (e.g., 30 to 40°C), it is preferred that some of the amino acids represented by X³ in the P sequence block are non-aromatic, hydrophobic amino acids, such as valine and leucine (preferably valine), and the other amino acids are aromatic, hydrophobic amino acids, such as phenylalanine and tryptophan (preferably phenylalanine). In this case, the proportion of the number of non-aromatic, hydrophobic amino acids represented by X³ relative to the total number of X³s in the P sequence block is, for example, 60 to 95%, preferably 70 to 80%, and more preferably 75 to 85%, and the proportion of the number of aromatic, hydrophobic amino acids represented by X³ relative to the total number of X³s in the P sequence block is, for example, 5 to 40%, preferably 20 to 30%, and more preferably 15 to 25%. Further, in this case, the aromatic, hydrophobic amino acids represented by X³ preferably appear as dispersed as possible in the P sequence block. For example, it is preferred that a repeat unit in which X³ represents an aromatic, hydrophobic amino acid appears at a ratio of one to, for example, two to eight repeat units (VPGX³G), preferably three to seven repeat units (VPGX³G) , more preferably four to six repeat units (VPGX³G), and even more preferably five repeat units (VPGX³G).
m represents an integer of 5 or more. m is preferably 10 to 100, more preferably 10 to 50, even more preferably 15 to 35, and particularly preferably 20 to 30.

The elastin-like block peptide sequence is not particularly limited as long as it comprises the G sequence block and the P sequence block. The number of G sequence blocks in the elastin-like block peptide sequence is not particularly limited, and is, for example, 1 to 5, preferably 2 to 5, more preferably 2 to 4, even more preferably 2 to 3, and particularly preferably 2. The number of P sequence blocks in the elastin-like block peptide sequence is not particularly limited, and is, for example, 1 to 5, preferably 1 to 4, more preferably 1 to 3, and even more preferably 1 to 2.

The blocks (the G sequence block and the P sequence block, the G sequence block and the G sequence block, and the P sequence block and the P sequence block) may be directly linked or may be linked via a linker sequence. Preferably, a linker sequence is interposed between the blocks.

The linker sequence is not particularly limited as long as it does not significantly reduce the fiber-forming ability resulting from the self-assembly of the elastin-like block peptide sequence, and usually any amino acid or amino acid sequence can be used without significant limitation. The linker sequence is, for example, 1 to 20, preferably 1 to 15, more preferably 1 to 10, even more preferably 1 to 8, and still even more preferably 2 to 8 amino acids in length. Specific examples of the linker sequence include the amino acid sequence set forth in SEQ ID NO: 7: LWLGSG and the amino acid sequence set forth in KL. Furthermore, amino acid sequences in which one or more (for example, one to five, preferably one to three, more preferably one or two, and even more preferably one) amino acids are mutated (for example, substituted, deleted, inserted, and/or added, preferably substituted, more preferably conservatively substituted) relative to any of these amino acid sequences can also be used.

In the present specification, "conservative substitution" means the substitution of an amino acid with another amino acid having a similar side chain. For example, the substitution between amino acids having a basic side chain, such as lysine, arginine, or histidine, is considered to be a conservative substitution. The following substitutions between other amino acids are also considered to be a conservative substitution: the substitution between amino acids having an acidic side chain such as aspartic acid or glutamic acid; the substitution between amino acids having an uncharged polar side chain, such as glycine, asparagine, glutamine, serine, threonine, tyrosine, or cysteine; the substitution between amino acids having a nonpolar side chain, such as alanine, valine, leucine, isoleucine, proline, phenylalanine, methionine, or tryptophan; the substitution between amino acids having a β-branched side chain, such as threonine, valine, or isoleucine; and the substitution between amino acids having an aromatic side chain, such as tyrosine, phenylalanine, tryptophan, or histidine.

From the viewpoint of the ability to form fibers by self-assembly, the sequence structure of the elastin-like block peptide sequence is preferably, from the N-terminal side, G sequence block-P sequence block-G sequence block, G sequence block-P sequence block-P sequence block-G sequence block, P sequence block-G sequence block, or G sequence block-P sequence block. The sequence structure is particularly preferably G sequence block-P sequence block-G sequence block or G sequence block-P sequence block-P sequence block-G sequence block. In these structures, "-" indicates a direct link (between blocks) or another sequence (e.g., a linker sequence).

The polypeptide of the present invention may comprise a cell adhesive sequence block at a terminus thereof. The cell adhesive sequence block is not particularly limited, and various sequences can be used. The cell adhesive sequence block may be one that does not have cell selectivity, such as GRGDS (SEQ ID NO: 9) or RGDS (SEQ ID NO: 10), or one that has cell selectivity (e.g., REDV (SEQ ID NO: 22)). Examples of sequences that have cell selectivity include HHH, VVV, TTT, TGA, NNN, KKK, AAA, RRR, YYY, TTT, GAT, GGG, PGH, GQA, QGD, GIG, EKG, KGK, QGF, GMK, GLS, CAG, CNG, KGT, PLG, NRG, CSG, LGL, AVG, GHP, GLI, GVG, GPS, SPG, GPP, GIS, GYL, GEK, QGE, CNY, FPG, GAP, APG, GEC, LPG, GPR, PCG, GDV, IGG, CDG, AVA, FLM, GFD, GTP, GPY, VSG, DGR, GIT, GFL, ASG, GCP, NQG, SGL, GGA, PDG, QAL, GLK, GSP, GEP, GNS, AKG, DGY, TGP, VGP, SLW, AAG, AGA, ARG, GRD, EGF, GSC, PGQ, HSQ, EAP, RGP, PGD, CNI, GFG, GPT, GDQ, KGE, PFI, QGP, SYW, LPGFPGLK (SEQ ID NO: 11), LPGFPGTP (SEQ ID NO: 12), GPPGLSGPP (SEQ ID NO: 13), FPGPPGPP (SEQ ID NO: 14), LPGLPGPP (SEQ ID NO: 15), FPGLPGPP (SEQ ID NO: 16), GPPGPPGSPG (SEQ ID NO: 17), LPGPPGPP (SEQ ID NO: 18), FPGSPGFPG (SEQ ID NO: 19), GSPGLPGTP (SEQ ID NO: 20), and IGLSGEKG (SEQ ID NO: 21).

When the polypeptide of the present invention comprises the cell adhesive sequence block, the cell adhesive sequence is positioned at a terminus or the termini (the N-terminus and/or the C-terminus) of the polypeptide of the present invention, preferably at the C-terminus. The cell adhesive sequence may be positioned at either the N-terminus or the C-terminus, or both. In a preferred embodiment of the present invention, the cell adhesive sequence is positioned at only one terminus.

When the polypeptide of the present invention comprises the cell adhesive sequence block, the elastin-like block peptide sequence and the cell adhesive sequence may be directly linked, or another amino acid sequence (e.g., a sequence that is 3 to 30, 7 to 25, or 10 to 20 amino acids in length) may be interposed between the two. The other amino acid sequence is preferably a hydrophilic amino acid-rich sequence from the viewpoint of cell adhesion. In other words, it is preferred that the hydrophilic amino acid-rich sequence is positioned on the side opposite to the end of the cell adhesive sequence. The hydrophilic amino acid-rich sequence is not particularly limited as long as it is a sequence in which the proportion of hydrophilic amino acids is high. Examples include amino acid sequences that are, for example, 3 to 15 or 5 to 10 amino acids in length and contain hydrophilic amino acids in an amount of, for example, 50% or more, 60% or more, 70% or more, 80% or more, or 90% or more. The hydrophilic amino acid-rich sequence is preferably a histidine consecutive sequence (His tag sequence) from the viewpoint of facilitating the purification of the polypeptide of the present invention. The other amino acid sequence preferably contains an amino acid having a functional group that can be used in a crosslinking reaction (e.g., lysine or cysteine).

The polypeptide of the present invention has an amino group. The amino group may be one possessed by an amino acid constituting the polypeptide of the present invention, or may be one added by chemical modification. The polypeptide of the present invention preferably comprises an amino acid residue having an amino group. Examples of an amino acid having an amino group include lysine, arginine, and histidine. Such an amino acid is preferably, for example, an amino acid in which a side chain is a hydrocarbon chain (e.g., an alkyl group, preferably an alkyl group having 1 to 8 carbon atoms) substituted with -NH³, and particularly preferably lysine.

It is thought that the amino group reacts with an aldehyde group of a water-soluble polymer described below to form a chemical bond, whereby the polypeptide of the present invention is linked with the water-soluble polymer via a covalent bond, further enhancing strength and/or bioadhesive properties after self-healing. While the polypeptide of the present invention is known to form a gel having self-healing properties, there was a concern that the linkage of another polymer with the polypeptide would change the structure of gel-forming fibers and also impair the self-healing properties; therefore, it was unexpected that the self-healing properties are not impaired even when the polypeptide of the present invention is used in combination with the water-soluble polymer.

When the polypeptide of the present invention comprises an amino acid residue having an amino group, the amino acid residue may be present within the elastin-like block peptide sequence (for example, within the P sequence block, linker, or the like) or may be present outside the elastin-like block peptide sequence. Preferably, the polypeptide of the present invention can comprise a crosslinking sequence block containing an amino acid residue having an amino group, separately from the other sequence blocks. The crosslinking sequence block can further enhance strength and/or bioadhesive properties after self-healing.

The crosslinking sequence block can contain preferably 2 or more, more preferably 2 to 10, even more preferably 2 to 5, and still even more preferably 2 to 3 amino acid residues each having an amino group. The proportion of the number of amino acid residues having an amino group relative to the total number of amino acid residues in the crosslinking sequence block is, for example, 20% or more, preferably 30% or more, more preferably 40% or more, and is, for example, 80% or less, preferably 70% or less, and more preferably 60% or less. In one preferred embodiment, the crosslinking sequence block is KAAK (SEQ ID NO: 8).

When the polypeptide of the present invention comprises the crosslinking sequence block, its position is not particularly limited. In one embodiment of the present invention, the crosslinking sequence block is preferably positioned on the C-terminal side of the elastin-like block peptide sequence, and when the cell adhesive sequence block is present, the crosslinking sequence block is preferably positioned between the elastin-like block peptide sequence and the cell adhesive sequence block.

The polypeptide of the present invention may be chemically modified as long as the thixotropic gel forming properties are not significantly impaired. The presence or absence of thixotropic gel forming properties can be evaluated according to the Examples or previous reports.

The polypeptide of the present invention may have a carboxyl group (-COOH), carboxylate (-COO⁻), amide (-CONH₂), or ester (-COOR) at the C-terminus.

"R" in the ester is, for example, a C₁₋₆ alkyl group, such as methyl, ethyl, n-propyl, isopropyl, or n-butyl; a C₃₋₈ cycloalkyl group, such as cyclopentyl or cyclohexyl; a C₆₋₁₂ aryl group, such as phenyl or α-naphthyl; a phenyl-C₁₋₂ alkyl group, such as benzyl or phenethyl; a C₇₋₁₄ aralkyl group, such as an α-naphthyl-C₁₋₂ alkyl group, such as α-naphthyl methyl; or a pivaloyloxymethyl group.

The polypeptide of the present invention may have an amidated or esterified carboxyl group (or carboxylate), which is not the carboxyl group at the C-terminus. The ester in this case may be, for example, the ester at the C-terminus described above.

The polypeptide of the present invention further encompasses polypeptides having the amino group of the N-terminal amino acid residue protected by a protective group (e.g., a C₁₋₆ acyl group, including C₁₋₆ alkanoyl, such as a formyl group and an acetyl group); polypeptides having the N-terminal glutamine residue pyroglutamated that can be formed due to cleavage in vivo; polypeptides having a substituent (e.g., -OH, -SH, an amino group, an imidazole group, an indole group, and a guanidino group) on a side chain of an amino acid in the molecule protected by an appropriate protective group (e.g., a C₁₋₆ acyl group, including a C₁₋₆ alkanoyl group, such as a formyl group and an acetyl group); conjugated proteins, such as glycoproteins to which a sugar chain is bonded; and the like.

The polypeptide of the present invention may be in the form of a pharmaceutically acceptable salt formed with an acid or base. The salt can be any pharmaceutically acceptable salt, and can be either an acid salt or a basic salt. Examples of acid salts include inorganic acid salts, such as hydrochloride, hydrobromide, sulfate, nitrate, and phosphate; organic acid salts, such as acetate, propionate, tartrate, fumarate, maleate, malate, citrate, methanesulfonate, and para-toluenesulfonate; and amino acid salts, such as aspartate and glutamate. Examples of basic salts include alkali metal salts, such as sodium salts and potassium salts; and alkaline-earth metal salts, such as calcium salts and magnesium salts.

The polypeptide of the present invention may be in the form of a solvate. The solvent can be any pharmaceutically acceptable solvent, and may be, for example, water, ethanol, glycerol, or acetic acid.

The polypeptide of the present invention can be easily produced according to or based on a known genetic engineering technique or according to or based on a known chemical synthesis method. For example, the production may be performed by using PCR, restriction enzyme cleavage, DNA ligation techniques, in vitro transcription/translation techniques, recombinant protein production techniques, solid-phase synthesis techniques, peptide-to-peptide ligation techniques, and the like.

The polypeptide of the present invention may be used singly or in a combination of two or more.

From the viewpoint of gel-forming properties, self-healing properties, and the like, the concentration of the polypeptide of the present invention in the composition of the present invention is, for example, 0.2 w/v% or more, preferably 0.4 w/v% or more, more preferably 0.5 w/v% or more, and is, for example, 3.0 w/v% or less, preferably 2.0 w/v% or less, more preferably 1.5 w/v% or less, and even more preferably 1.2 w/v% or less.

The water-soluble polymer is not particularly limited as long as it is a polymer having an aldehyde group and is water-soluble.

The water solubility is not particularly limited as long as the composition of the present invention can be formed into a gel when water is used as a solvent. The water solubility is such that, for example, the solubility in 100 g of water at 23°C is, for example, 1 g or more, preferably 2 g or more, more preferably 5 g or more, and even more preferably 10 g or more.

The viscosity-average molecular weight of the polymer is not particularly limited and is, for example, 5000 to 300000. From the viewpoint of strength and/or bioadhesive properties after self-healing, the viscosity-average molecular weight is preferably 10000 to 200000, more preferably 15000 to 150000, even more preferably 20000 to 100000, still even more preferably 25000 to 80000, particularly preferably 30000 to 60000, and most preferably 30000 to 50000. The viscosity-average molecular weight can be measured by the most appropriate method according to the type of polymer.

The number of aldehyde groups is not particularly limited and is, for example, from 0.1 to 100 per 1000 of the viscosity-average molecular weight of the polymer. The number is preferably from 0.5 to 70, more preferably from 1 to 50, even more preferably from 2 to 30, still even more preferably from 3 to 20, and particularly preferably from 5 to 15, per 1000 of the viscosity-average molecular weight of the polymer. The number of aldehyde groups can be measured by the most appropriate method according to the type of polymer. For example, when the polymer is obtained by a method of oxidizing a polysaccharide to generate aldehyde groups in sugar residues, the number of aldehyde groups can be calculated by measuring the degree of oxidation.

The structural units (constituent monomers) of the water-soluble polymer are not particularly limited. The water-soluble polymer preferably comprises a sugar as a structural unit. In this case, the number of sugars (sugar residues) per 100 structural units is preferably 50 or more, more preferably 70 or more, further preferably 80 or more, still more preferably 90 or more, and particularly preferably 95 or more. The water-soluble polymer is particularly preferably a polysaccharide or an oxidized polysaccharide.

Oxidized polysaccharides can be produced according to or based on known methods. For example, an oxidized polysaccharide can be obtained by reacting a polysaccharide in the presence of a periodate.

The type of sugar as a structural unit, the structure of the polymer (linear or branched), and the types of linkage between the structural units (for example, in the case of a polysaccharide, α-linkage, β-linkage, and the carbon numbers involved in linkage formation) are not particularly limited. In a preferred embodiment of the present invention, the sugar as a structural unit is glucose, the structure of the polymer is branched, and the type of linkage between the structural units is an α-linkage (in particular, α-1,6-linkage and α-1,4-linkage). The water-soluble polymer is particularly preferably oxidized dextran and/or oxidized dextrin.

The water-soluble polymer is preferably a water-soluble polymer having no charge.

The water-soluble polymer may be used singly or in a combination of two or more.

The concentration of the water-soluble polymer in the composition of the present invention is, for example, 0.5 w/v% or more, preferably 1.0 w/v% or more, more preferably 1.5 w/v% or more, and even more preferably 2.0 w/v% or more, and is, for example, 10.0 w/v% or less, preferably 7.0 w/v% or less, more preferably 5.0 w/v% or less, and even more preferably 4.0 w/v% or less, from the viewpoint of gel-forming properties, self-healing properties, and the like.

The composition of the present invention may comprise other components. Examples of other components include cells and drugs.

The composition of the present invention may be in dry form or wet form. From the viewpoint of immediate usability as a medical device or the like, the composition of the present invention is preferably a gel composition or a gel-forming sol composition. In this case, for example, the composition of the present invention can comprise a conjugate of the polypeptide of the present invention and the water-soluble polymer linked via a covalent bond, and the polypeptide of the present invention can self-assemble into nanofibers. Preferably, the composition of the present invention comprises a conjugate of nanofibers formed of the polypeptide of the present invention and the water-soluble polymer linked via a covalent bond.

The composition of the present invention can be produced by various methods. From the viewpoint of, in particular, the ease of obtaining the composition of the present invention in gel form or gel-forming sol form having self-healing properties, the composition of the present invention is preferably produced by a method comprising mixing a sol composition converted from a gel composition comprising the polypeptide of the present invention with a water-soluble polymer having an aldehyde group.

The gel composition comprising the polypeptide of the present invention can be obtained by dissolving the polypeptide of the present invention in a solvent at a temperature lower than the LCST (e.g., 0 to 15°C, 2 to 10°C), heating the resulting solution to the LCST or higher (e.g., 30 to 50°C, 30 to 45°C, 30 to 40°C), and allowing the solution to stand for a specific period of time (e.g., 1 to 300 hours, preferably 8 to 120 hours, more preferably 12 to 80 hours). As the solvent, water is used. A mixed solvent of water and an organic solvent can also be used. A monosaccharide, such as sucrose, is preferably added (for example, at a final concentration of 2 to 20 w/v%, 5 to 15 w/v%) to the solvent.

The sol composition converted from the gel composition can be obtained by applying mechanical strain (e.g., pipetting, vortexing, or ultrasonic stirring) to the gel composition. The sol composition is considered to have gel-forming ability since it forms a gel when the application of mechanical strain is eliminated or weakened.

The water-soluble polymer having an aldehyde group is preferably dissolved in a solvent, such as water, before mixing. It is preferable that a monosaccharide, such as sucrose, is added (for example, at a final concentration of 2 to 20 w/v%, 5 to 15 w/v%) to the solvent.

The ratio of the volume of the sol composition to the volume of the water-soluble polymer solution is, for example, 1 to 10, preferably 2 to 6, and more preferably 3 to 5.

The method for mixing the sol composition and the water-soluble polymer is not particularly limited, and mixing may be performed by, for example, pipetting, vortexing, ultrasonic stirring, or the like.

After mixing, as described above, the mixture is allowed to stand at a temperature equal to or higher than the LCST for a specific period of time, thereby obtaining the composition of the present invention in gel form.

The composition of the present invention can be used for various applications and can be, in particular, suitably used for medical devices. Therefore, in one embodiment, the present invention relates to a medical device comprising the composition of the present invention (the medical device of the present invention).

The medical device is not particularly limited as long as it is a machine, an instrument, or the like intended for use in the diagnosis, treatment, or prevention of diseases in humans or animals, or intended to affect the structure or function of the body of humans or animals.

The target organisms are not particularly limited, and examples include mammals, such as humans, monkeys, dogs, cats, horses, cows, pigs, sheep, mice, rats, and rabbits, as well as vertebrates, including these.

The composition of the present invention is in gel form or is capable of forming a gel. The composition of the present invention can be converted into a sol when mechanical strain is applied, and can be reconverted into a gel when the application of mechanical strain is stopped. Therefore, the composition of the present invention can be applied to a target to be protected in liquid form, and is suitable for use as a material that promotes healing while reinforcing a target site to be protected by gelation. From this viewpoint, the composition of the present invention is preferably used for medical devices for use in protecting or adhering to an internal sutured site, a cut surface of an organ or tissue, or a damaged site.

The sutured site may be inside or outside the body, and is preferably an internal sutured site. More specifically, the sutured site is, for example, a sutured site in a gastrointestinal tract, a blood vessel, a nerve, a bile duct, a pancreatic duct, a ureter, skin, or the like, and is preferably a sutured site in a gastrointestinal tract. Protecting a sutured site is not particularly limited as long as protection is applied to the sutured site and the periphery thereof. More specifically, protecting a sutured site means physically reinforcing the sutured site to prevent disruption. This makes it possible to suppress or prevent anastomotic leakage.

The "cut surface" refers to a cut surface formed when a part of an organ or tissue is resected. Examples of the organ or tissue include the pancreas, liver, kidney, blood vessel, lung, thyroid, and prostate. Protecting the cut surface is not particularly limited as long as protection is applied to the cut surface to enable suppression or prevention of the leakage of body fluids from the cut surface. In one embodiment, the composition of the present invention can be used, for example, by being applied to a cut surface of an organ, such as the spleen, to adhere a nearby tissue (adipose tissue, intestinal tract, etc.) thereto, thereby forming a patch on the cut surface.

The damaged site may be inside or outside the body. The portion of the damaged site is in accordance with the sutured site or the cut surface described above. Protecting the damaged site is not particularly limited as long as protection is applied to the cut surface to enable suppression or prevention of the leakage of body fluids from the cut surface.

The composition of the present invention can be used by being applied or sprayed onto a target to be protected in sol form. The composition of the present invention can form a gel even under conditions in which mechanical strain comparable to that of a living body in motion is applied. Due to such excellent properties, the composition of the present invention can stay at a target site to be protected and exerts a protective function.

### Examples

The present invention is described below in detail with reference to Examples; however, the present invention is not limited to these Examples.

### 1. Experimental Procedure

### 1.1. Production of Oxidized Dextran (OD)

Dextran (Dextran 40,000 (viscosity-average molecular weight: 40,000), Wako Pure Chemical Industries, Ltd., Japan) was dissolved in 50 mL of ultrapure water, and 1.6 g of sodium periodate (Wako Pure Chemical Industries, Ltd., Japan) as an oxidizing agent was added thereto, followed by stirring in a dark room for 3.5 hours. To stop the oxidation reaction, 0.6 mL of glycerol (Wako Pure Chemical Industries, Ltd., Japan) was added, and the mixture was further stirred for 1 hour. The solution was placed in a dialysis membrane (Spectra/Por (registered trademark) 1 Dialysis Membrane, MWCO 6-8000, Spectrum Laboratories, Inc., U.S.A.) and dialyzed against 2 L of ultrapure water at 4°C. The ultrapure water was exchanged three times every 24 hours. Subsequently, the solution was frozen at -80°C and freeze-dried using a freeze dryer (FDU-1200, Tokyo Rikakikai Co., Ltd., Japan) to obtain a powdery sample.

### 1.2. Evaluation of OD

FT-IR spectra of dextran and the OD produced in Section 1.1 were obtained using a Fourier transform infrared spectrometer (FT/IR-610, JASCO, Japan). Each sample and potassium bromide (Wako Pure Chemical Industries, Ltd., Japan) were weighed at a ratio of 1:49 and mixed using a mortar and pestle to obtain measurement samples, and spectroscopic measurement was performed in accordance with the KBr method.

### 1.2.1. Hydroxylamine Hydrochloride (HA-HCl) Titration

To measure the degree of oxidation of the OD produced in Section 1.1, titration was performed using HA-HCl. HA-HCl (Tokyo Chemical Industries, Ltd., Japan), NaOH (Nacalai Tesque, Inc., Japan), and methyl orange (Wako Pure Chemical Industries, Ltd., Japan) were individually dissolved in ultrapure water to prepare a 0.25 M HA-HCl aqueous solution, a 1 M NaOH aqueous solution, and a 0.1 mg/mL methyl orange aqueous solution, respectively. 100 mg of the OD was dissolved in 25 mL of the HA-HCl aqueous solution and shaken at room temperature for 3 hours. A few drops of the methyl orange aqueous solution were added as an indicator, and titration was performed with the NaOH aqueous solution until the color reached that of the HA-HCl aqueous solution in which the OD was not dissolved. The degree of oxidation was calculated according to the following equation.$\begin{matrix}Degree of Oxidation \left(%\right) \\ = \frac{Titration volume of NaOH \left(mL\right) \times Concentration of NaOH \left(M\right)}{\frac{Mass of OD \left(mg\right)}{Molar mass of the repeat unit \left(g/mol\right)} \times 2 \left(the mumber of aldehyde groups in the repeat unit\right)} \times 100\end{matrix}$

### 1.3. Production of GPG-KR/OD Conjugated Hydrogel

### 1.3.1. Synthesis of Polypeptide (GPG-KR)

The following polypeptide (GPG-KR) was synthesized: a polypeptide (SEQ ID NO: 6) in which a crosslinking sequence (SEQ ID NO: 8: KAAK) and a cell adhesive sequence (SEQ ID NO: 9: GRGDS) were positioned on the C-terminal side of a polypeptide (SEQ ID NO: 5) comprising an elastin-like block peptide sequence that comprises a G sequence block consisting of (SEQ ID NO: 3: VGGVG)₅, a P sequence block consisting of (SEQ ID NO: 4: VPGXG)₂₅, wherein each X is the same or different, and represents V or F, a G sequence block consisting of (SEQ ID NO: 3: VGGVG)₅ in this order from the N-terminal side, each block being linked via a linker sequence. GPG-KR is a polypeptide consisting of a previously reported amino acid sequence.

Specifically, E. *coli* BLR(DE3) strain was transformed with plasmid DNA encoding the polypeptide, and the polypeptide was expressed. The polypeptide was obtained by purification using metal ion affinity chromatography using an His-tag. SDS-PAGE and MALDI-TOF-MS confirmed that the target polypeptide was obtained.

The recovered peptide solution was placed in Spectra/Por (registered trademark) 1 Dialysis Membrane, MWCO 6-8000 (Spectrum Laboratories), followed by dialysis against 2 L of ultrapure water at 4°C. Ultrapure water was exchanged twice every hour (minimum time), then twice every 2 hours, and then once after 1.5 hours, a total of five times, for the dialysis. After the dialysis, the peptide solution was filtered through Minisart (registered trademark) Syringe Filter, 0.2 µm (Sartorius Stedim Biotech), attached to a syringe to remove any insolubilized peptide, followed by freezing at -80°C overnight. Thereafter, freeze drying was performed with an FDU-1200 freeze dryer (Tokyo Rikakikai) to obtain GPG-KR powder.

### 1.3.2. Production of GPG-KR Hydrogel

Sucrose (Wako Pure Chemical Industries, Ltd., Japan) was dissolved in ultrapure water to prepare a 100 mg/mL aqueous sucrose solution. To this aqueous solution used as a solvent, GPG-KR was added in an ice bath, and the mixture was shaken at 4°C for 6 hours for complete dissolution. After adjusting the peptide concentration to 10 mg/mL using a NanoDrop 2000, the solution was allowed to stand at 45°C for 3 days to cause gelation.

### 1.3.3. Production of GPG-KR/OD Conjugated Hydrogel

The OD produced in Section 1.1 was dissolved in a 100 mg/mL aqueous sucrose solution to prepare a 150 mg/mL OD solution. The GPG-KR gel produced in Section 1.3.2 was liquefied by vortex shaking and subjected to ultrasonic stirring (40 kHz, 5 min). Thereafter, the two solutions, i.e., the GPG-KR solution and the OD solution, were mixed at a ratio of GPG-KR solution:OD solution of 4:1 and subjected again to ultrasonic stirring (40 kHz, 5 min). The resulting solution was allowed to stand at 45°C for 3 days, thereby producing a conjugated gel. The produced sample was denoted as "GPG-KR_OD," and a sample obtained by using a 100 mg/mL aqueous sucrose solution instead of the OD solution was denoted as "GPG-KR."

### 1.4. Evaluation of GPG-KR/OD Conjugated Hydrogel

### 1.4.1. Sodium Dodecyl Sulfate-polyacrylamide Gel Electrophoresis (SDS-PAGE)

The GPG-KR hydrogel and the GPG-KR/OD conjugated hydrogel were each individually dissolved in ultrapure water at 4°C, and 20 µM solutions were prepared by measuring the absorbance at 280 nm using a NanoDrop 2000 (Thermo Fisher Scientific Inc., U.S.A.). The molar extinction coefficients of the GPG-KR hydrogel and the GPG-KR/OD conjugated hydrogel were both 11380 dm³/(mol·cm).

6 g of sodium dodecyl sulfate (SDS; Wako Pure Chemical Industries, Ltd., Japan), 15 mL of glycerol, 25 mg of CBB R-250 (Wako Pure Chemical Industries, Ltd., Japan), 0.91 g of tris(hydroxymethyl)aminomethane (SERVA Electrophoresis GmbH, Germany), and 40 mL of ultrapure water were mixed, and the pH was adjusted to 7.0 with hydrochloric acid. Thereafter, ultrapure water was added to reach a final total volume of 50 mL. The resulting product was denoted as "Sample Buffer."

A mixture of 6 µL of Unstained Protein Standard, Broad Range (10-200 kDa) (New England Biolabs, Inc., U.K.) and 6 µL of Sample Buffer was used as a marker, and a mixture of 18 µL of an ELP aqueous solution and 6 µL of Sample Buffer was used as a sample. These mixtures were allowed to stand at 37°C for 30 minutes. A polyacrylamide precast gel for electrophoresis, i.e., e-PAGEL (E-T 12.5L), was attached to an electrophoresis tank. An anode buffer obtained by adjusting the pH of a 0.1 mol/L tris(hydroxymethyl)aminomethane solution to 8.9 using 1 mol/L hydrochloric acid, as well as a cathode buffer comprising 0.1 mol/L tris(hydroxymethyl)aminomethane, 0.1 mol/L tricine (Dojindo Laboratories, Japan), and 1.0 w/v% SDS, were poured into the electrophoresis tank. After the marker and the sample were loaded onto the wells of the gel, electrophoresis was performed at 35 mA for about 30 minutes. The electrophoresis tank used was an AE-6500 Rapidus Mini-Slab electrophoresis tank (ATTO CO., Japan). Thereafter, the gel was removed and stained by immersion for about 1 hour in a mixed solution of 250 mL of methanol (Kanto Chemical Co., Inc., Japan), 50 mL of acetic acid (Kanto Chemical Co., Inc., Japan), 0.5 g of CBB R-250, and 200 mL of ultrapure water while mixing by inverting the container. The gel was removed from the solution and then destained by immersion in a mixed solution of 180 mL of methanol, 50 mL of acetic acid, and 170 mL of ultrapure water for about 2 hours.

### 1.4.2. Nuclear Magnetic Resonance (NMR) Spectroscopy

The ¹H nuclear magnetic resonance spectrum of the hydrogel prepared in heavy water was measured using a nuclear magnetic resonance spectrometer (Ascend 500, AVANCE NEO, Bruker, USA). A high-resolution magic angle spinning (HR-MAS) probe was used as the probe, and the number of scans was 64.

### 1.4.3. Shear Flow and Recovery Behavior Test

To examine the recovery behavior of the produced samples, a shear flow and recovery behavior test was performed using a rheometer (MCR302 Modular Compact Rheometer, Anton Paar GmbH, Austria). The measurement was performed using a 1° cone- and-plate (diameter: 25 mm) at a gap of 0.048 mm. To prevent drying of the samples during the measurement, Prowipes moistened with ultrapure water were placed around the measurement stage, followed by covering with a solvent trap cover. Furthermore, the temperature was maintained at 37°C using the rheometer's built-in Peltier system.

After loading the samples, a time sweep measurement was performed for 1 hour at a constant strain of 1% and a constant frequency of 1 Hz in order to recover the structure disrupted by lowering the fixture.

Thereafter, the recovery behavior of the gel was evaluated by sequentially applying a high shear strain (100%) for 60 seconds and a low shear strain (0.5%) for 600 seconds over 3 cycles at a constant frequency of 1 Hz.

### 1.4.4. Lap Shear Measurement

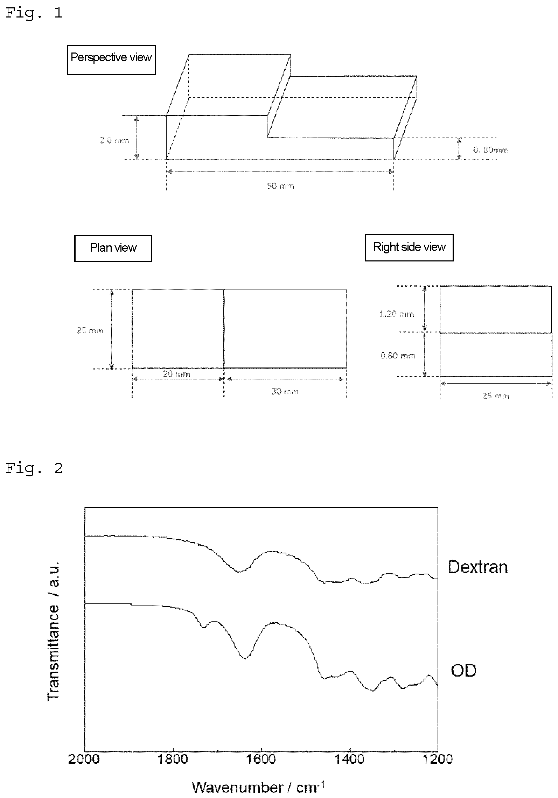

A lap shear measurement was performed to examine the bioadhesive properties of the produced samples. The test method conformed to ASTM F2255-05. An aluminum mold was used as the test specimen, and porcine small intestine was used as the adherend substrate. Fig. 1 shows the mold shape.

Thawed porcine small intestine (Tokyo Shibaura Zouki Co., Ltd.) was washed with phosphate buffered saline (PBS) and cut into pieces with a size of 25 mm × 30 mm. The resulting pieces were adhered to the test specimens using a cyanoacrylate adhesive (Toagosei Co., Ltd.). The gel liquefied by vortex shaking was applied to an end portion of the small intestine over a width of 10 mm, and the test specimens were bonded to each other such that the small intestine portions overlapped. Thereafter, the test specimens were covered with Prowipes moistened with PBS, wrapped in plastic wrap, and allowed to stand at 37°C for 2 hours. As a comparative target, test specimens in which small intestines were adhered using an existing tissue adhesive (Beriplast (registered trademark)) were also produced.

The lap shear measurement was performed using a tensile testing machine (Autograph AGS-50NX, Shimadzu Co., Ltd., Japan). The test was performed three times for each sample under conditions of a crosshead speed of 5 mm/min. The test results were expressed as "test force (N)" and "stroke (mm)," and the maximum value of the test force was converted into shear strength (kPa) according to the following equation.$Shear strength \left(kPa\right) = \frac{Maximum test force \left(N\right)}{Area in which the sample was applied \left({mm}^{2}\right)} \times {10}^{3}$

### 1.4.5. In vivo Gelation Test

The in vivo gelation behavior of the samples was examined through in vivo testing. Black mice anesthetized with 4% isoflurane underwent a midline laparotomy, and the gel liquefied by vortex shaking (300 µL for GPG-KR, 250 µL for GPG-KR_OD) was injected into the rectouterine pouch. After closing the abdomen, the mice were maintained in a supine position for 10 minutes and were allowed to move freely after recovery from anesthesia. A second laparotomy was performed after 24 hours to observe the progress.

### 2. Results

### 2.1. Evaluation of OD

### 2.1.1. FT-IR Spectroscopic Measurement

Fig. 2 shows the results of the FTIR spectroscopic measurement performed on the produced samples. The OD exhibited a peak at around 1730 cm⁻¹ originating from the C=O double bond, which was not observed in dextran, confirming that some of the hydroxy groups were oxidized to aldehyde groups.

### 2.1.2. HA-HCl Titration

HA-HCl titration was performed on the produced sample, resulting in a titration volume of 9.9 mL. By substituting this value into the equation described in Section 1.2.1, the degree of oxidation of the OD was determined to be 79.9%.

### 2.2. Evaluation of GPG-KR/OD Conjugated Hydrogel

### 2.2.1. Sodium Dodecyl Sulfate-polyacrylamide Gel Electrophoresis (SDS-PAGE)

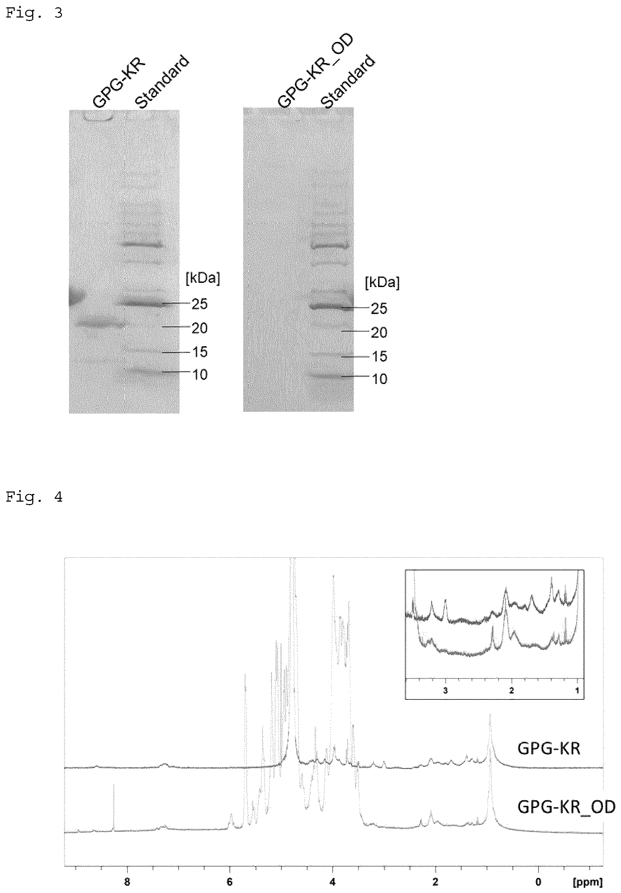

Fig. 3 shows the results of SDS-PAGE performed on the produced samples. According to the observation results for GPG-KR, stained bands were observed at the origin of the electrophoresis lane where the sample was loaded and at approximately 20 kDa. The band observed at the origin of the electrophoresis lane corresponds to GPG nanofibers that self-assembled to form high-molecular weight aggregates, and the stained band observed at approximately 20 kDa corresponds to the GPG-KR monomer of approximately 18 kDa. In GPG-KR_OD, these stained bands were not observed. Since CBB binds to the amino groups of proteins, it was suggested that in GPG-KR_OD, the amino groups of GPG-KR may form chemical bonds with the OD.

### 2.2.2. Nuclear Magnetic Resonance Spectroscopy (NMR)

Fig. 4 shows the results of the NMR spectroscopic measurement performed on the produced samples. The measurement results confirmed that in GPG-KR_OD, the peaks at 3.0 ppm and 1.7 ppm originating from the εCH2 and δCH2 of lysine residues in GPG-KR disappeared.

### 2.2.3. Shear Flow and Recovery Behavior Test

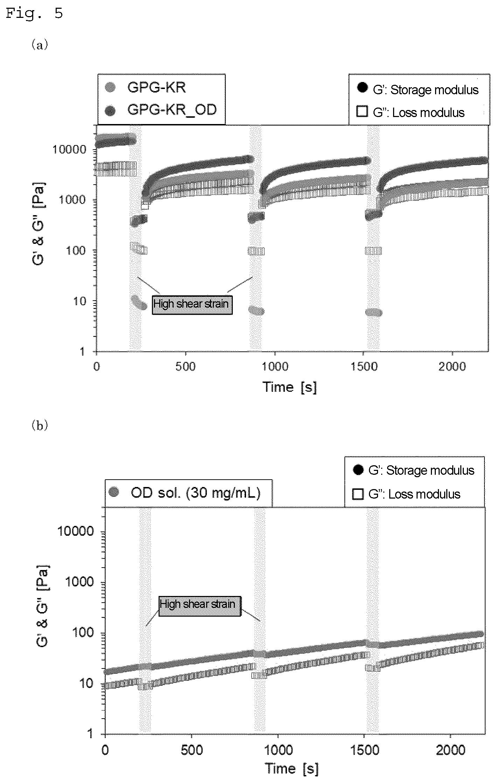

Fig. 5 shows the results of the recovery behavior test performed on the produced samples. According to the measurement results, GPG-KR and GPG-KR_OD exhibited viscous behavior in which G" exceeded G' under 100% high shear strain, and returned to elastic behavior in which G' again exceeded G" when the high shear strain was released. In a 30 mg/mL OD solution, G' always exceeded G", and the above behavior was not observed. The recovery rate of the storage modulus was calculated by dividing the value of the storage modulus before the application of high shear strain by the value of the storage modulus after the third relaxation time. According to the calculation results, the recovery rate was 12.9% for GPG-KR and 41.5% for GPG-KR_OD, indicating an improvement in self-healing properties.

### 2.2.4. Lap Shear Measurement

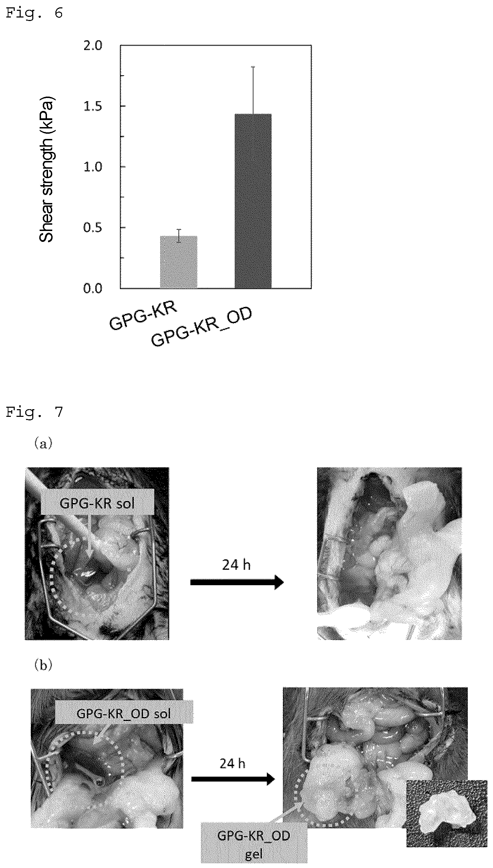

Fig. 6 shows the results of the lap shear measurement performed on the produced samples. The measurement results revealed that GPG-KR/OD exhibited higher shear strength than GPG-KR, indicating an improvement in bioadhesive properties.

### 2.2.5. In vivo Gelation Test

Fig. 7 shows the results of the in vivo gelation test performed on the produced samples. For GPG-KR, gelation of the sample could not be confirmed after 24 hours, and the material had disappeared. For GPG-KR/OD, the sample formed a gel after 24 hours, and adhesion to the reproductive organs was observed.

## Claims

1. A composition comprising:
a polypeptide that comprises an elastin-like block peptide sequence and has an amino group, the elastin-like block peptide sequence comprising a G sequence block consisting of (SEQ ID NO: 1: X¹GGX²G)ₙ, wherein each X¹ is the same or different, and represents V or L, each X² is the same or different, and represents V or L, and n represents an integer of 4 or more, and a P sequence block consisting of (SEQ ID NO: 2: VPGX³G)ₘ, wherein each X³ is the same or different, and represents any amino acid, and m represents an integer of 5 or more; and
a water-soluble polymer having an aldehyde group.

2. The composition according to claim 1, wherein the composition is a gel composition or a gel-forming sol composition.

3. The composition according to claim 1, wherein the polypeptide comprises an amino acid residue having an amino group.

4. The composition according to claim 3, wherein the polypeptide comprises a crosslinking sequence block containing two or more amino acid residues each having an amino group.

5. The composition according to claim 1, wherein the polypeptide comprises a cell adhesive sequence block.

6. The composition according to claim 1, wherein the water-soluble polymer comprises a sugar as a structural unit.

7. The composition according to claim 6, wherein the water-soluble polymer is an oxidized polysaccharide.

8. The composition according to claim 6, wherein the water-soluble polymer is oxidized dextran and/or oxidized dextrin.

9. The composition according to claim 1, wherein the composition comprises a conjugate of the polypeptide and the water-soluble polymer linked via a covalent bond.

10. A method for producing the composition according to any one of claims 1 to 9, comprising:
(a) mixing a sol composition converted from a gel composition with a water-soluble polymer having an aldehyde group, the gel composition comprising a polypeptide that comprises an elastin-like block peptide sequence and has an amino group, the elastin-like block peptide sequence comprising a G sequence block consisting of (SEQ ID NO: 1: X¹GGX²G)ₙ, wherein each X¹ is the same or different, and represents V or L, each X² is the same or different, and represents V or L, and n represents an integer of 4 or more, and a P sequence block consisting of (SEQ ID NO: 2: VPGX³G)ₘ, wherein each X³ is the same or different, and represents any amino acid, and m represents an integer of 5 or more.

11. A medical device comprising the composition according to any one of claims 1 to 9.

12. The medical device according to claim 11 for use in protecting or adhering to a sutured site, a cut surface of an organ or tissue, or a damaged site.
